(19) Europäisches Patentamt
European Patent Office
Office européen des brevets

(11) Publication number: **0 317 561**
**B1**

(12) **EUROPEAN PATENT SPECIFICATION**

(45) Date of publication of patent specification: **30.01.91**

(51) Int. Cl.$^5$: **C 07 D 249/20,**
D 06 M 13/352, D 06 P 1/62

(21) Application number: **87904766.0**

(22) Date of filing: **29.06.87**

(86) International application number:
**PCT/US87/01554**

(87) International publication number:
**WO 88/00942 11.02.88 Gazette 88/04**

# (54) SULFONATE BENZOTRIAZOLES AND THEIR USE IN POLYAMIDE.

(30) Priority: **28.07.86 US 889705**

(43) Date of publication of application:
**31.05.89 Bulletin 89/22**

(45) Publication of the grant of the patent:
**30.01.91 Bulletin 91/05**

(84) Designated Contracting States:
**AT BE CH DE FR GB IT LI NL SE**

(56) References cited:
**EP-A-0 006 564**
**WO-A-40/2365**

**Chemical Abstracts, volume 77, no. 6, 7 August 1972, (Columbus, Ohio, US), see page 98, abstract 36262r**

(73) Proprietor: **ALLIED-SIGNAL INC.**
**Columbia Road and Park Avenue P.O. Box 2245R**
**Morristown New Jersey 07960 (US)**

(72) Inventor: **HARRIS, Paul, Wesley**
**2321 Hey Road**
**Richmond, VA 23224 (US)**
Inventor: **HOPF, Frederick, Robert**
**350 Parsippany Road**
**Parsippany, NJ 07054 (US)**
Inventor: **YARDLEY, James, Thomas**
**40 Macculloch Avenue**
**Morristown, NJ 07960 (US)**

(74) Representative: **Brock, Peter William et al**
**URQUHART-DYKES & LORD 91 Wimpole Street**
**London W1M 8AH (GB)**

**EP 0 317 561 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to sulfonated hydroxy benzotriazoles and their use to improve stain resistance and dye lightfastness in nylon fibers. Particularly useful is a new sulfonated 2-(2'-hydroxyaryl)-2H-benzotriazole.

The unsulfonated, precursor 2-(2'-hydroxyaryl)-2H-benzotriazole (hereinafter sometimes called hydroxy benzotriazole or aryl benzotriazole) is disclosed for use to protect organic substances from light-induced deterioration in U.S.—A—4,226,763 and U.S.—A—4,278,589. Use of sulfonated 2-(2'-hydroxyaryl)-2H-benzotriazoles as photostabilizing agents for natural and synthetic fibers is disclosed in WO 84/02365.

Surprisingly, it has been found that certain sulfonated aryl benzotriazoles also improve resistance of synthetic nylon fibers to staining by common anionic stain agents, such as food dyes containing Acid Red (Kool Aid®), Kool Aid is a soft drink produced by General Foods and is very widely available in the USA, and these sulfonated aryl benzotriazoles improve lightfastness of dye on the nylon fiber. Some of these sulfonated aryl benzotriazoles are new compounds, namely, compounds having the structure

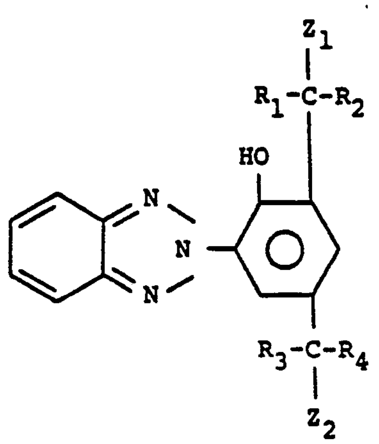

wherein $R_1$, $R_2$, $R_3$ and $R_4$ are independently the same or different alkyl groups and $Z_1$ and $Z_2$ are alkyl or sulfonated aryl group, provided at least $Z_1$ or $Z_2$ is sulfonated aryl group.

Mixtures of the compounds of the structure given above can be used to treat fibers to improve resistance to staining by anionic staining compounds such as Acid Red food dyes used in Kool Aid and also improve lightfastness of dyes on nylon fibers. The fibers can be treated with the sulfonated hydroxy benzotriazoles of the structures given above, preferably in aftertreatment wherein the treatment is at a preferred pH of between about 2 to 5.

The method for improving resistance of nylon to staining by anionic staining compounds and to improve lightfastness of dyes on nylon comprises depositing 0.05 to 5 percent by weight of a sulfonated 2-(2'-hydroxyaryl)-2H-benzotriazole on the nylon, by treating the nylon, at a temperature between 0 and 100°C, wtih an aqueous solution of sulfonated hydroxy benzotriazole selected from

I

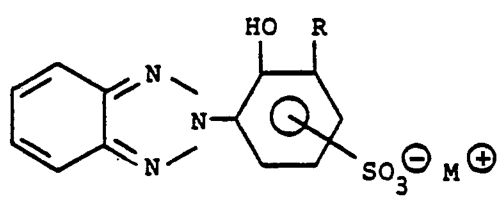

II

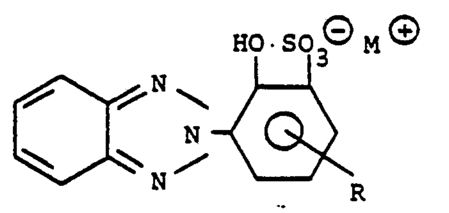

where R = tertiary alkyl or tertiary aralkyl, and M is a positive ion, such as alkali metal or hydrogen,

III

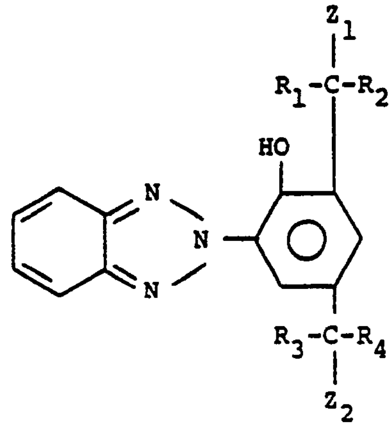

where $R_1$, $R_2$, $R_3$, $R_4$, $Z_1$ and $Z_2$ are as defined above and

IV

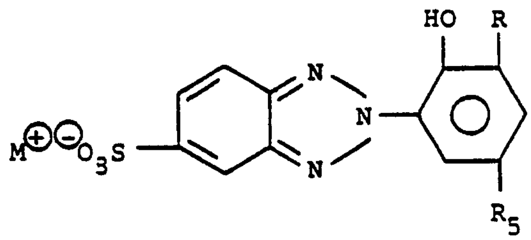

where M is a positive ion, such as alkali metal or hydrogen, R is tertiary alkyl or tertiary aralkyl and $R_5$ is a short chain alkyl.

The preferred method of treatment is an aftertreatment at a pH of between 2 and 5. The preferred treatment temperature is between 30°C and 90°C. The preferred time to aftertreat the fiber is after dyeing, preferably from 10 to 30 minutes in duration. The compounds I, III and IV are also useful to improve properties of nylon materials in other forms, such as film.

This invention relates to the preparation of novel chemical compounds amnd to novel methods of application to nylon (polyamide) materials (particularly polyamide fibers) which may or may not contain dyes wherein (a) the nylon materials and any incorporated dyes are afforded significant reduction in photodegradation and/or (b) the nylon materials are afforded significant improvement in resistance to many common stains, especially stains involving anionic species (such as FD & C Red 40, a common foodstuff dye). The novelty of the invention lies in (a) the chemical composition of certain of the additives which have not been previously reported and which provide significant and surprising photostability to polyamides and to dyes in polyamides at very low loadings relative to traditional photostabilizing additives, and (b) the method of application which provides for effective and durable incorporation into polyamides from aqueous solution under mild temperature conditions.

Traditional agents employed to enhance photostability of dyed polyamides include compounds of structures such as

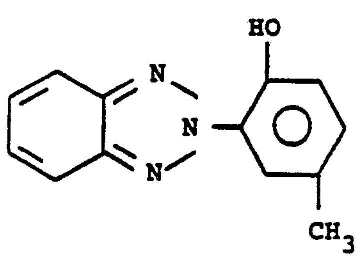

Tinuvin® P

;

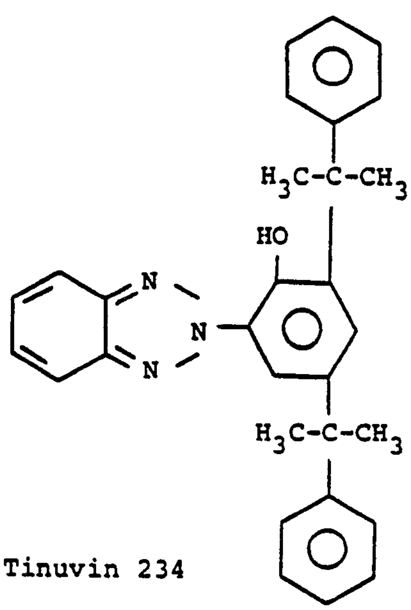

Tinuvin 234

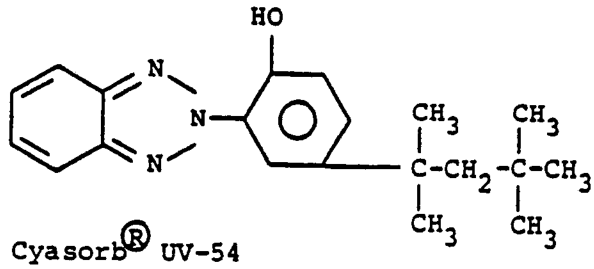

Cyasorb® UV-54

Since these compounds are generally soluble only in organic solvents, the incorporation of such material into nylon poses serious problems. Surface application from organic solvents is a hazardous process and may result in a non-uniform coating of poor durability. Addition of these materials during extrusion may result in thermal degradation, may result in an ineffective distribution of material, and may result in loss of material due to volatility under extruder conditions.

We have found that compounds of the general structure

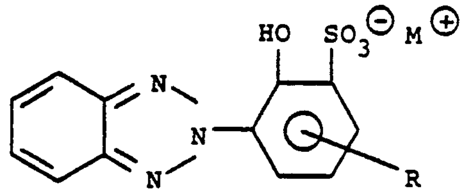

where M is a positive cation, such as an alkali metal or hydrogen and R is tertiary alkyl or tertiary aralkyl may be effectively incorporated into nylon fiber from aqueous solution at relatively low pH (preferably pH $<3$) and at modest temperatures (0°C—100°C, preferably 20—70°C). Nylon fibers or dyed nylon fibers treated with this material (0.05—5 percent, preferably 0.1—1 percent by weight) either before or after dyeing show dramatically enhanced photostability relative to untreated materials. In addition, these fibers demonstrate increased resistance to staining by certain common stains, especially those containing anionic groups.

In addition, we have discovered that compounds of the general structures

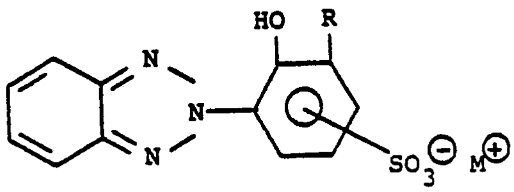

where R = tertiary alkyl or tertiary aralkyl, and particularly sulfonated T-234 (ST-234), which is a mixture of

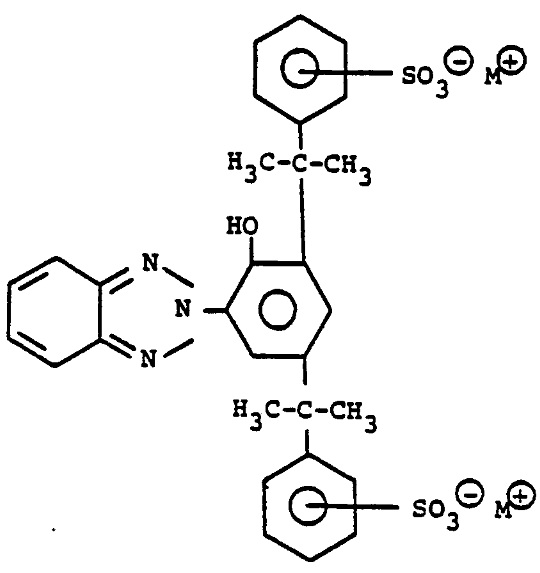

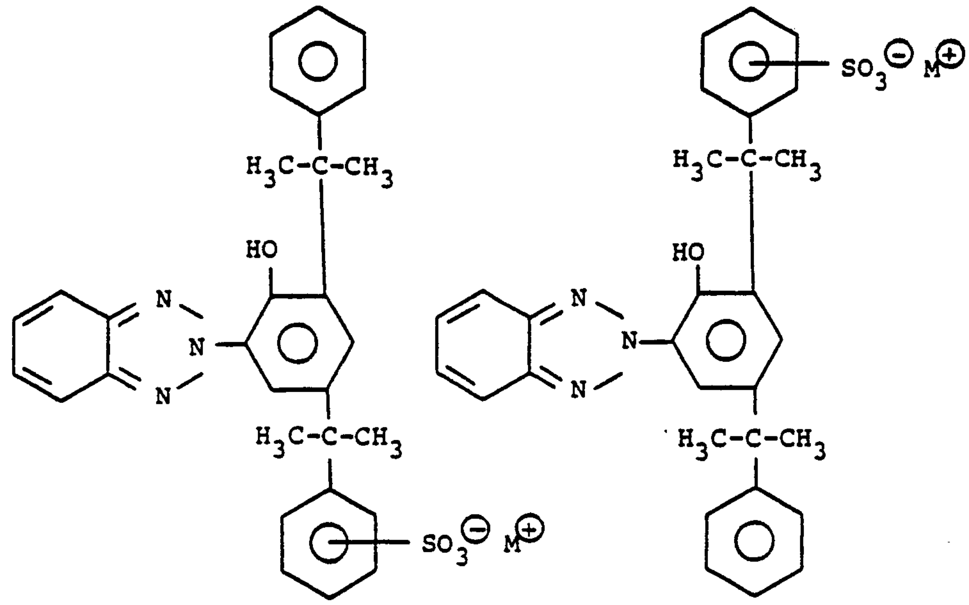

where M is an alkali metal or hydrogen, and the $SO_3^{\ominus}M^{\oplus}$ group is para or ortho afford a surprising increase of stain resistance of nylon fiber and lightfastness of dye on nylon fiber.

Example I. Synthesis of STP

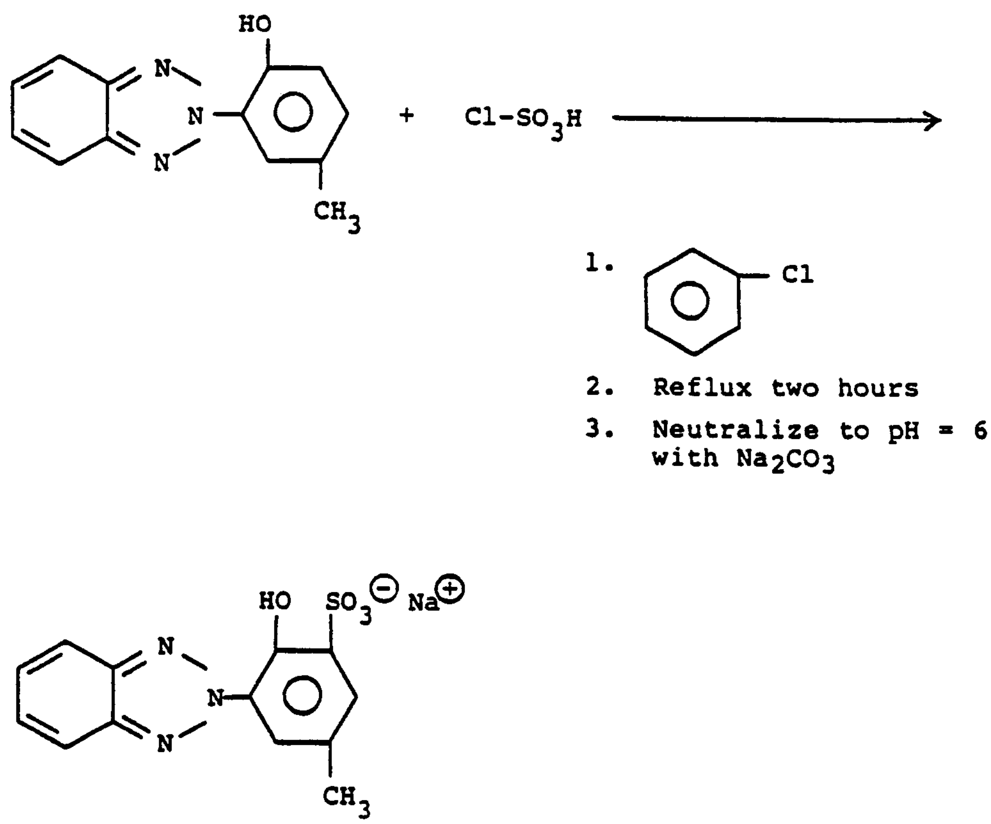


A solution of 53.5 grams (0.238 mole) of Tinuvin-P in 650 milliliters of chlorobenzene was added to a 1 liter round bottom one-neck flask equipped with a magnetic stirrer and heating mantle and set up for reflux under an atmosphere of nitrogen. Then 15.85 milliliters (0.238 mole) of reagent grade chlorosulfonic acid was slowly added to the stirred solution over a period of approximately twenty minutes. The resulting

mixture was brought to reflux and maintained at reflux for 1½ hours. The solution was allowed to cool to room temperature under a slow stream of nitrogen. The contents of the flask were poured, with stirring, into a 2-liter beaker containing 300 grams of crushed ice.

The resulting emulsion was neutralized to pH = 6.0 with 10 percent aqueous sodium carbonate solution. The solid product was filtered on a large Buchner funnel, using a medium speed filter paper. After several hours of air drying on the funnel, the resulting precipitate was washed with three 250-milliliter portions of toluene. The washed product was air-dried for 10 hours. This nearly dry crude product was recrystallized from 4.5 liters of boiling water which had a pH = 5 (adjusted with sulfuric acid). The precipitate was allowed to settle at room temperature for 12 hours, then cooled to approximately 5°C in a refrigerator for three hours.

The resulting, finely divided, nearly colorless crystals were filtered on a coarse fritted glass funnel, and air dried for several hours. Actual yield of dry product was 51.6 grams, which is 71.2 percent of the theoretical yield.

Example II. Synthesis of ST-234

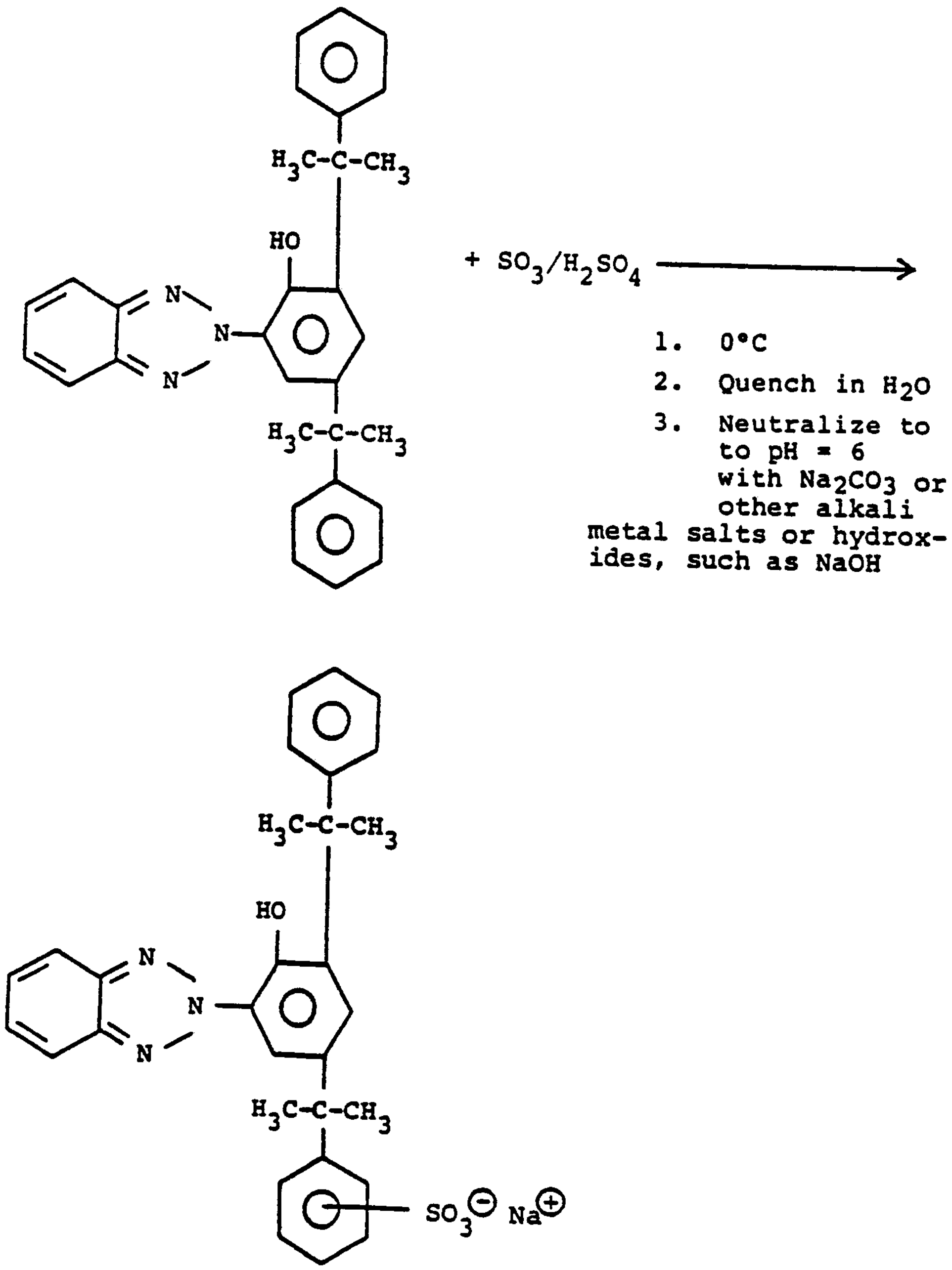

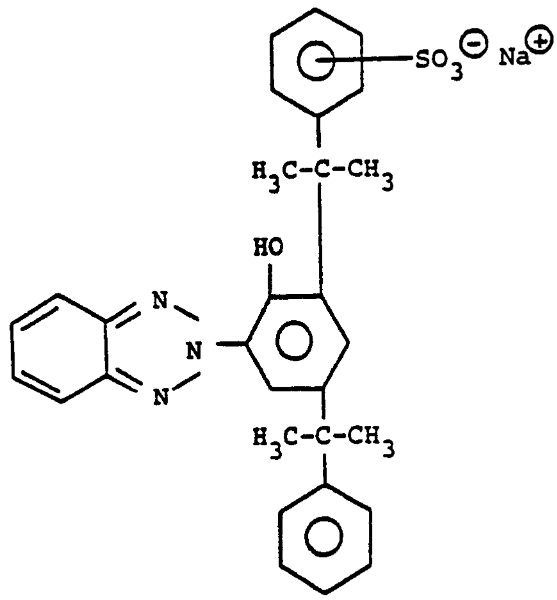

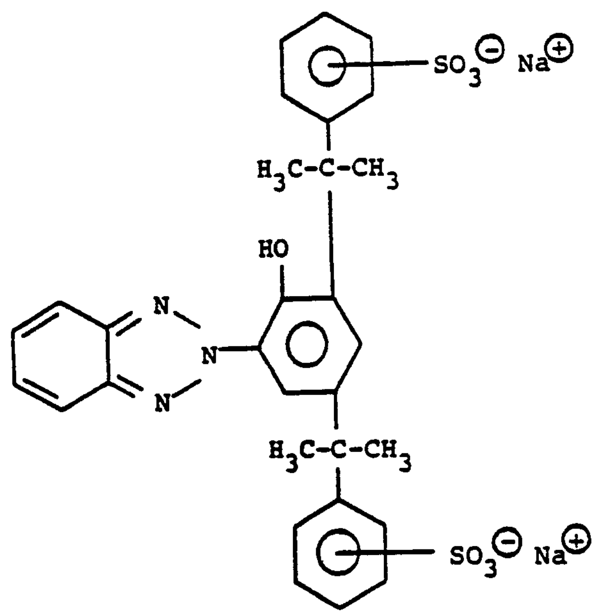

A solution of 5 percent fuming sulfuric acid was prepared by adding 2.3 milliliters of 20 percent fuming sulfuric acid to 6.8 milliliters of concentrated sulfuric acid (0.0105 mole of $SO_3$). then 4.47 grams (0.0100 mole) of powdered Tinuvin-234 were slowly added to the vigorously stirred solution of fuming sulfuric acid which was maintained at a temperature below 25°C. Most of the Tinuvin-234 went into solution. The small residue of undissolved solid was allowed to stir at room temperature for about one hour until complete dissolution.

The yellow reaction mixture was quenched in 50 milliliters of ice water. The resultant mixture was neutralized to pH = 7 with saturated aqueous sodium carbonate solution. The resultant total volume was approximately 150 milliliters. This mixture was allowed to stand and settle for 12 hours. The precipitate was collected on a coarse glass fritted disc filter and washed three times with 20-milliliter portions of cold water. The precipitate was vacuum dried at 50°C for 12 hours. The weight of dried product is nearly the theoretical amount, but contains a small percentage of coprecipitated sodium sulfate. This material was used to treat nylon for improved lightfastness without further purification.

Example III.

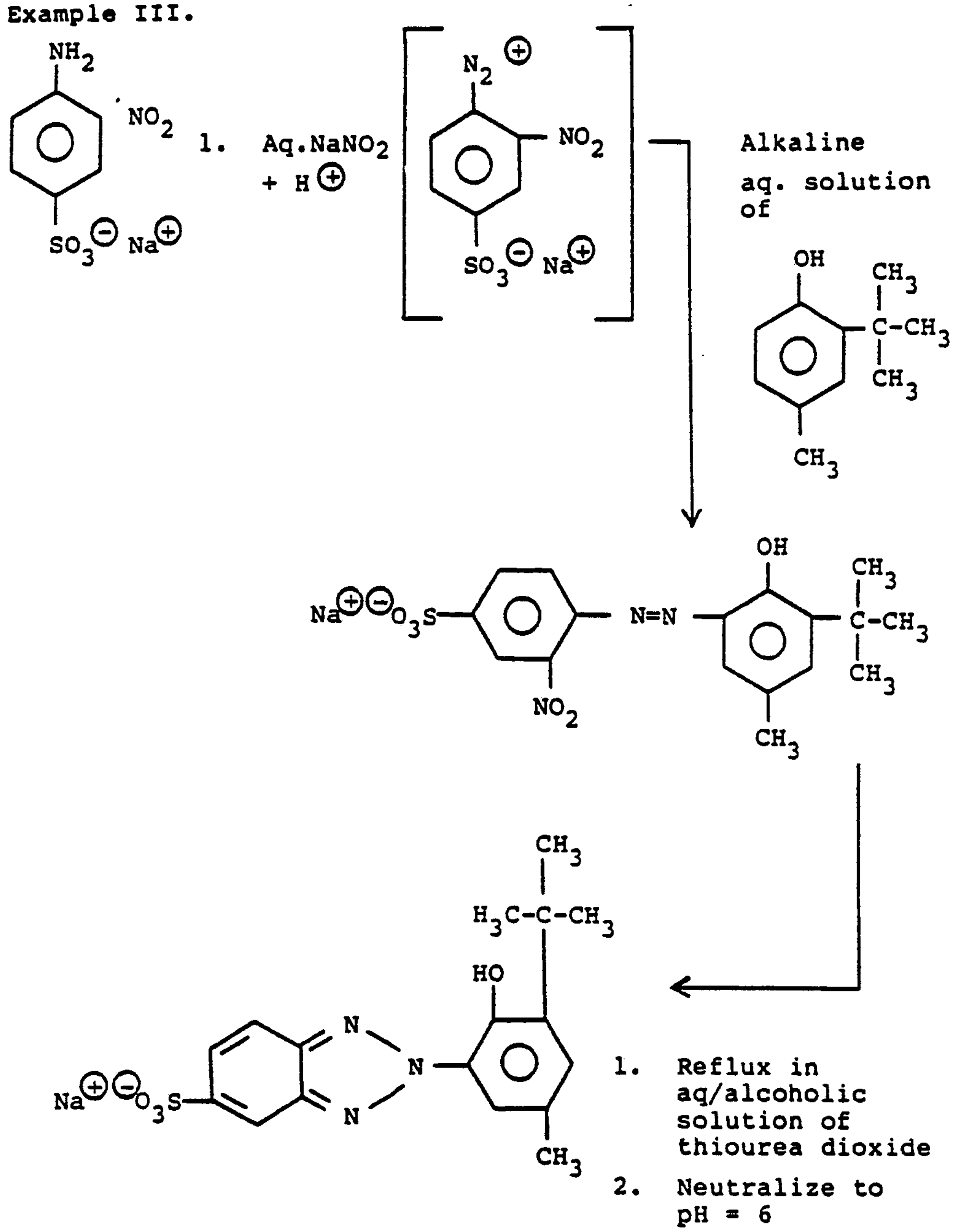


Example IV

Photostability of UV Light Screens Dissolved in Polymer Film Matrices

| Light Screen Agent | Polymer | Destruction, % * |
|---|---|---|
| Tinuvin P | PMMA ** | < 9 |
| Tinuvin P | Nylon-6 | >65 |
| STP | PMMA | < 9 |
| STP | Nylon-6 | >60 |
| Tinuvin 234 | PMMA | < 7 |
| Tinuvin 234 | Nylon-6 | < 5 |
| ST-234 | Nylon-6 | < 5 |

* Twenty-one hours of irradiation in a Rayonet Photochemical Reactor equipped with RPR-3000 lamps at temperatures of 38 - 40°C and ambient humidity. Amount of destruction determined by ultraviolet spectrophotometry.

** Polymethylmethacrylate.

This example demonstrates that hydroxybenzotriazoles lose photostability in a polyamide environment. It also demonstrates that incorporation of bulky hydrophobic groups near the intramolecular hydrogen bond (especially in the ortho position relative to the hydroxyl) effectively provides for a high degree of photostability.

Typical Application Procedure

Beakers containing from 0.5 to 2.0 percent (OWF) of STP or ST-234 or other soluble UV screen with a 20:1 liquid ratio and adjusted to the proper pH were heated to 71°C in a water bath (water bath had equilibrated at 71°C before adding "dye" beakers). Samples of nylon knitted sleeve were added (usually 1 or 2 samples, each weighing approximately 5 grams) and stirred constantly for 30 minutes. The sleeves were removed after 30 minutes and rinsed with distilled water. After rinsing, the sleeves were padded with paper towels to remove excess water. The damp sleeves were then placed in an oven (~100°C) for one hour. The dried samples were then allowed to equilibrate under ambient conditions, in the dark for at least 12 hours before irradiation testing.

Irradiation

Samples were suspended in a Rayonet RPR-100 Photochemical Reactor manufactured by The Southern New England Ultraviolet Company. For all of the irradiation testing reported, the reactor was fitted with 16 RPR-3000 lamps. The major output of these lamps is centered at 300 nanometers, with a significant 254 nanometers component and small amounts of radiation of longer wavelengths. No attempts were made to filter the output or restrict exposure to a specific bandwidth. During all irradiations, the internal air circulation fan was operating. This resulted in operating temperatures of approximately 38 to 40°C. No attempt was made to control humidity.

The samples were suspended at the midpoint of the sources and rotated on a turntable to assure uniformity of irradiation. To further assure uniformity of exposure, the samples were rotated equally between direct front and back surface exposure. On longer term irradiations, the samples were cycled several times between ambient dark conditions and ambient irradiation conditions.

Example V

Protective Effects of Water-Soluble UV Light Screens on Undyed Nylon-6 Knitted Sleeves

| Additive* | pH | Uptake, % ** | Color Before Irradiation | Color After Irradiation *** |
|---|---|---|---|---|
| Control | - | - | White | Yellow |
| STP | 2 | >95 | White | Light Yellow |
| ST-234 | 2 | >95 | White | Slightly Yellow |
| STP | 5 | 25 | Light Yellow | Yellow |
| ST-234 | 5 | 35 | White | Slightly Yellow |

* Applied at 71°C from 20:1 liquor ratio bath. Nominally loading 0.1 - 2.0%.

** Percent bath exhaustion.

*** Irradiation for 12 hours and for 40 hours in Rayonet Photochemical Reactor with RPR-3000 Lamps at 38 - 40°C temperature and ambient humidity.

This example illustrates that uptake and color of nylon sleeves treated with sulfonated UV light screens is vastly superior for application at pH = 2 relative to application at pH = 5. Also, the superior performance of ST 234 to STP is demonstrated.

Example VI.

Protective Effects of Water-Soluble UV Light Screens on Dyed* Nylon-6 Knitted Sleeves

| Additive** | pH | Uptake,% *** | Color Before Irradiation | Color After Irradiation**** |
|---|---|---|---|---|
| Control | - | - | Normal | Severely Faded |
| STP | 2 | >95 | Slightly Off-Shade | Good Protection |
| ST-234 | 2 | >95 | Normal | Excellent Protection |
| STP | 5 | 25 | Off-Shade Yellow Coloration | Good Protection |
| ST-234 | 5 | 40 | Normal | Excellent Protection |

* Grey dyeing was with the following dyes at the following conditions.

| OWF, % (1) | |
|---|---|
| 0.0115 | Tectilon Orange 3G (100% strength) (C.I. Acid Orange 156) |
| 0.0121 | Tectilon Red 2B (100% strength) (C.I. Acid Red 361) |
| 0.0135 | Telon Blue BRL (200% strength) (C.I. Acid Blue 234) |

(1) On weight of fabric)

Dyeing Conditions:
1% Dowfax 2A1
2% monosodium phosphate
pH 7 adj. w/trisodium phosphate
Boil 30 minutes

** Applied at 71°C from 20:1 liquor ratio, 0.1 - 2.0% (OWF) loading on non-DSR knitted sleeves. Similar results are obtained at room temperature.

*** Percentage bath exhaustion, by spectrophotometric determination.

**** After 12 hours irradiation in a Rayonet Photochemical Reactor with RPR-3000 lamps at temperatures of 38 - 40°C and ambient humidity.

This example demonstrates that UV screens which are sulfonated and which possess a bulky hydrophobic group near the intra-molecular H-bond (such as ST-234) may be effectively applied to dyed nylon sleeves and that such materials provide superior light screening capability compared to similar compounds without such a hydrophobic group (i.e., STP).

Best Mode

The compounds useful for this invention can be applied as an aftertreatment to dyed fibers, such as carpet face fibers, the preferred compounds are the ones labeled ST-234 above. In use a mixture of the mono- and disulfonated, both para and ortho isomers, are used. The compound may be applied in the aftertreatment either alone or in combination with other compounds, particularly those compounds which enhance stain resistance of the fibers, such as condensation product of formaldehyde with a diphenyl ether (hereinafter called FDE), and more fully disclosed in copending U.S. Serial No. *904,433* filed *September 8, 1987, corresponding to WO 88/02040.*

The recommended aftertreatment conditions for ST-234 are: 0.1 to 0.5 percent on weight of fabric (OWF) concentration in the aftertreatment bath, pH 2.1 (with citric acid), bath temperature 140°F (60°C), liquor to goods ratio 25:1, fabric time in bath 20 minutes.

Example VII

The mixture of compounds labeled ST-234 above was used in an aftertreatment bath to treat nylon carpet fiber previously dyed to a silver commercial carpet fiber color. Bath conditions were as set forth above except pH was 3.0, liquor to goods ratio was 30:1 and 0.47 percent (OWF) was applied. Lightfastness was improved over the control (no aftertreatment) to 1.45 ΔE from 4.12 ΔE for the control, or to an average Gray Scale rating of 3.5 at 120 SFU compared to control at 1.33 Gray Scale rating.

Silver dyeing was with the following dyes at the following conditions.

| OWF, % (1) | |
|---|---|
| 0.0104 | Tectilon Orange 3G (100% strength) (C.I. Acid Orange 156) |
| 0.0054 | Telon Red BRCL (250% strength) proprietary (Mobay) |
| 0.0126 | Telon Blue BRL (200% strength) (C.I. Acid Blue 324) |

(1) On weight of fabric

Dyeing Conditions:
- 1% Dowfax 2Al
- 2% monosodium phosphate
- pH 7 adj. w/trisodium phosphate
- Boil 30 minutes

Example VIII
(Staining Improvement)

Using the recommended aftertreatment bath conditions given above, except temperature was 110°F (43°C), the mixture of compounds labeled ST-234 above, or the compound labeled STP above, either alone or with the stain resistance enhancer FDE described above, were added to the aftertreatment bath for nylon carpet fiber in circular knitted sleeve form, predyed to the silver color described above. After treatment, stains were created with Cherry Kool Aid®, containing FD & C40 food coloring, by forcing about 5 cubic centimeters of Cherry Kool Aid® into the fabric of the knitted sleeve of carpet fiber and blotting after five minutes. Results are given in the table below. Stain rating is on a 0 to 10 scale used by trained observers, unaware of which carpet fiber sample sleeve was treated with which compound. In the scale, low numbers mean good stain resistance and vice versa.

| | | Lightfastness | | |
|---|---|---|---|---|
| Additive | OWF, % | ΔE 120 AFU | Gray Scale* 120 AFU | Stain Rating |
| None (Control) | 0 | 4.12 | 1.33 | 5 |
| FDE/STP | 2/0.5 | 2.52 | 3.67 | 0.75 |
| FDE/ST-234 | 2/0.5 | 1.70 | 3.67 | 0.75 |
| FDE | 2 | 3.49 | 3.17 | 0.75 |
| STP | 0.5 | 2.47 | 3.17 | 3.5 |
| ST-234 | 0.5 | 2.35 | 3.00 | 3.5 |

* Average, by AATCC 16E

Thus, it can be seen that STP and ST-234 compound improved the stain resistance considerably over the control, but that best stain resistance is dependent on use of FDE, with or without STP or ST-234 compounds. However, the best overall combination of properties considering both lightfastness and stain resistance is the combination of FDE with ST-234 which had an ΔE rating of only 1.7, and average Gray Scale reading of 3.67. Low ΔE numbers and high Gray Scale numbers mean less fading.

**Claims**

1. A sulfonated 2-(2'-hydroxyaryl)-2H-benzotriazole having the formula

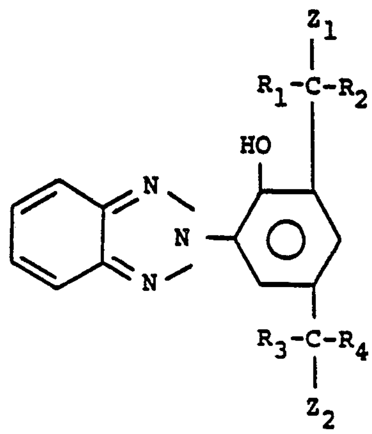

wherein $R_1$, $R_2$, $R_3$ and $R_4$ are independently the same or different alkyl groups and $Z_1$ and $Z_2$ are alkyl or sulfonated aryl group, provided at least $Z_1$ or $Z_2$ is sulfonated aryl group.

2. A method for improving resistance of nylon to staining by anionic staining compounds and improving light fastness of dyes on nylon characterised by depositing 0.05 to 5 percent by weight of a sulfonated 2-(2'-hydroxyaryl)-2H-benzotriazole on the nylon, by treating the nylon at a temperature between 0 and 100°C with an aqueous solution of sulfonated 2-(2'-hydroxyaryl)-2H-benzotriazole at mild temperatures selected from the group consisting of

I

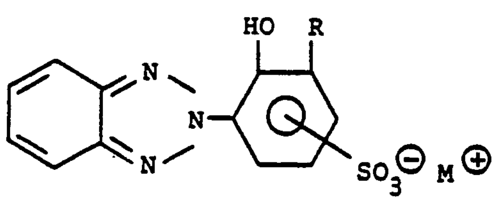

II

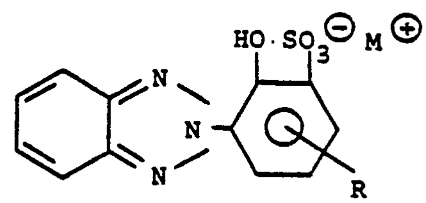

where R = tertiary alkyl or tertiary aralkyl, and M is a positive ion,

III

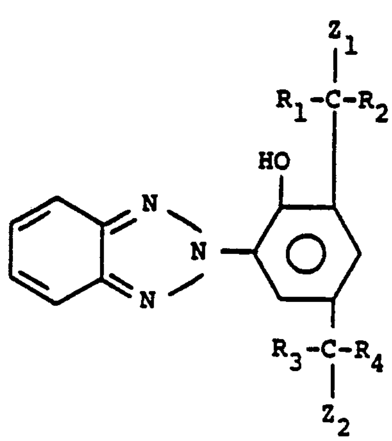

wherein $R_1$, $R_2$, $R_3$ and $R_4$ are independently the same or different alkyl groups and $Z_1$ and $Z_2$ are alkyl or sulfonated aryl group, provided at least $Z_1$ or $Z_2$ is sulfonated aryl group, and

IV

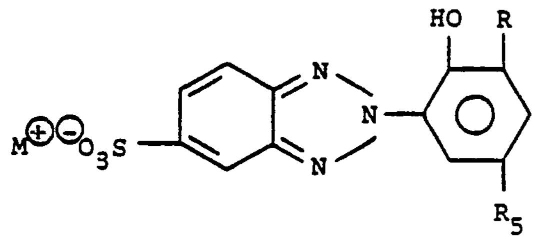

where M is a positive ion, R is tertiay alkyl or tertiary aralkyl, and $R_5$ is a short chain alkyl.

3. A method according to Claim 2 characterised in that the nylon is a fiber.

4. A method according to Claim 2 characterised in that the compound I, III, or IV is used to treat a nylon material other than fiber.

5. A method according to any one of Claims 2 to 4 characterised in that the said treatment is carried out at a pH between 2 and 5.

6. A method according to any one of Claims 2 to 5 characterised in that the temperature is from 30 to 90°C.

7. A method according to any one of Claims 2 to 6 characterised in that the treatment is carried out after dyeing of said nylon.

8. A method according to any one of Claims 2 to 7 characterised in that the treatment is carried out for between 10 and 30 minutes.

**Patentansprüche**

1. Ein sulfoniertes 2-(2'-hydroxyaryl)-2H-Benzotriazol mit der Formel

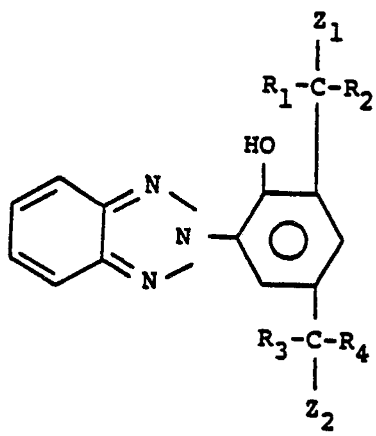

worin $R_1$, $R_2$, $R_3$ und $R_4$ unabhängig voneinander gleiche oder unterschiedliche Alkylgruppen und $Z_1$ und $Z_2$ Alkyl- oder sulfonierte Arylgruppen darstellen, unter der Maßgabe, daß zumindest $Z_1$ oder $Z_2$ eine sulfonierte Arylgruppe ist.

2. Verfahren zur Erhöhung des Widerstandes von Nylon gegen Fleckenbildung, hervorgerufen durch anionische fleckenbildende Verbindungen und zur Verbesserung der Lichtechtheit von Farben auf Nylon, dadurch gekennzeichnet, daß 0,05 bis 5 Gew.-% eines sulfonierten 2-(2'-hydroxyaryl)-2H-Benzotriazols auf dem Nylon abgelagert werden, durch Behandlung des Nylons bei einer Temperatur zwischen 0° und 100°C mit einer wässerigen Lösung des sulfonierten 2-(2'-hydroxyaryl)-2H-Benzotriazols bei milden Temperaturen, ausgewählt aus der Gruppe bestehend aus

I

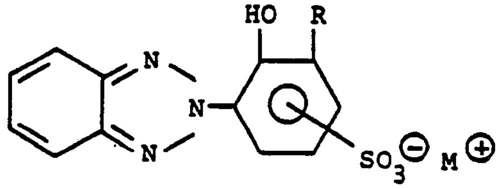

II

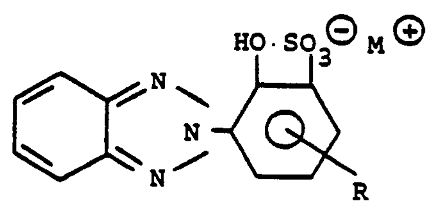

worin R= tertiäres Alkyl oder tertiäres Aralkyl und M ein positives Ion ist,

III

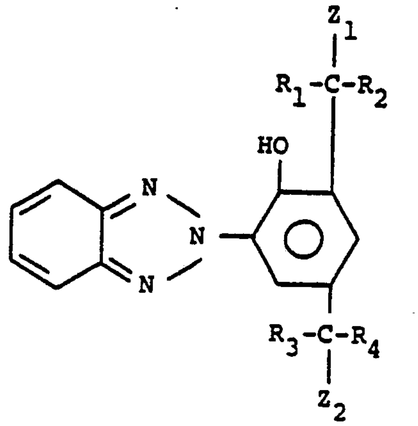

worin $R_1$, $R_2$, $R_3$ und $R_4$ unabhängig voneinander gleiche oder unterschiedliche Alkylgruppen und $Z_1$ und $Z_2$ Alkyl- oder sulfonierte Arylgruppen sind, unter der Maßgabe, daß zumindest $Z_1$ oder $Z_2$ eine sulfonierte Arylgruppe ist, und

IV

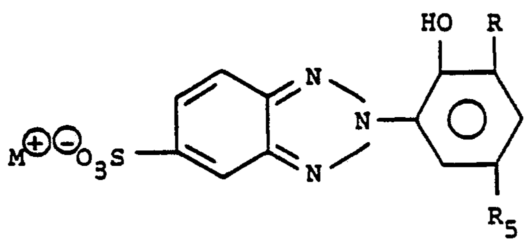

worin M ein positives Ion, R ein tertiäres Alkyl oder tertiäres Aralkyl, und $R_5$ ein kurzkettiges Alkyl ist.

3. Verfahren nach Anspruch 2, dadurch gekennzeichnet, daß das Nylons eine Faser ist.

4. Verfahren nach Anspruch 2, dadurch gekennzeichnet, daß die Verbindung I, III oder IV verwendet wird, um ein Nylonmaterial zu behandeln, das keine Faser ist.

5. Verfahren nach einem der Ansprüche 2 bis 4, dadurch gekennzeichnet, daß die Behandlung bei einem pH-Wert zwischen 2 und 5 durchgeführt wird.

6. Verfahren nach einem der Ansprüche 2 bis 5, dadurch gekennzeichnet, daß die Temperatur zwischen 30 und 90°C beträgt.

7. Verfahren nach einem der Ansprüche 2 bis 6, dadurch gekennzeichnet, daß die Behandlung nach dem Färben des Nylons durchgeführt wird.

8. Verfahren nach einem der Ansprüche 2 bis 7, dadurch gekennzeichnet, daß die Behandlung in 10 bis 30 Minuten durchgeführt wird.

**Revendications**

1. 2-(2'-hydroxyaryl)-2H-benzotriazole sulfoné répondant à la formule:

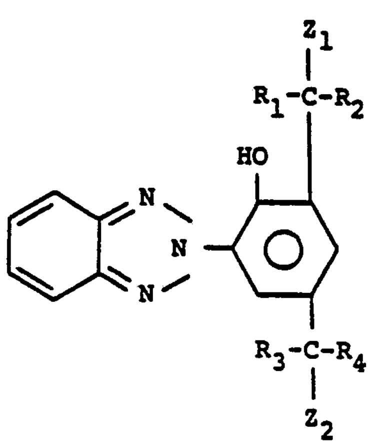

dans laquelle $R_1$, $R_2$, $R_3$ et $R_4$ sont indépendamment des groupes alkyle identiques ou différents et $Z_1$ et $Z_2$ sont des groupes alkyle ou des groupes aryle sulfoné, sous réserve qu'au moins un des radicaux $Z_1$ ou $Z_2$ est un groupe aryle sulfoné.

2. Procédé pour améliorer la résistance du nylon au tachage par des composés de tachage anioniques et pour améliorer la solidité à la lumière des colorants sur nylon, caractérisé en ce qu'on dépose sur le nylon 0,05 à 5% en poids d'un 2-(2'-hydroxyaryl)-2H-benzotriazole sulfoné, en traitant le nylon à une température comprise entre 0 et 100°C par une solution aqueuse d'un 2-(2'-hydroxyaryl)-2H-benzotriazole sulfoné choisi parmi

I

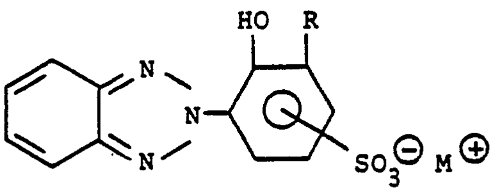

II

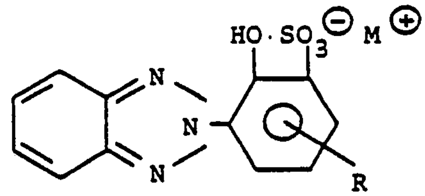

où R est un groupe alkyle tertiaire ou aralkyle tertiaire et M est un ion positif,

III

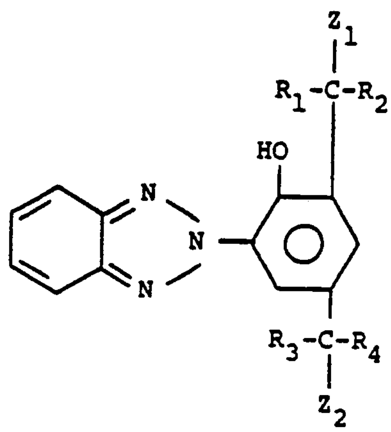

où $R_1$, $R_2$, $R_3$ et $R_4$ sont indépendamment des groupes alkyle identiques ou différents et $Z_1$ et $Z_2$ sont des groupes alkyle ou des groupes aryle sulfoné, sous réserve qu'au moins un des radicaux $Z_1$ ou $Z_2$ est un groupe aryle sulfoné, et

IV

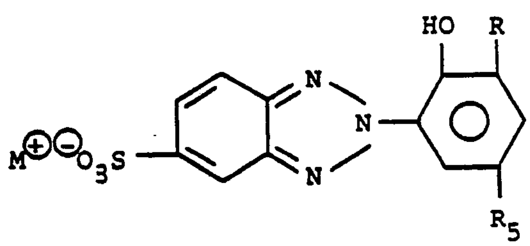

où M est un ion positif, R est un groupe alkyle tertiaire ou aralkyle teritaire, et $R_5$ est un groupe alkyle à chaîne courte.

3. Procédé selon la revendication 2, caractérisé en ce que le nylon est un fibre.

4. Procédé selon la revendication 2, caractérisé en ce qu'on utilise les composés I, III ou IV pour traiter une matière de nylon autre qu'une fibre.

5. Procédé selon l'une quelconque des revendications 2 à 4, caractérisé en ce que ce traitement est effectué à un pH compris entre 2 et 5.

6. Procédé selon l'une quelconque des revendications 2 à 5, caractérisé en ce que la température est de 30 à 90°C.

7. Procédé selon l'une quelconque des revendications 2 à 6, caractérisé en ce que le traitement est effectué après teinture de ce nylon.

8. Procédé selon l'une quelconque des revendications 2 à 7, caractérisé en ce que le traitement est effectuée pendant une durée comprise entre 10 et 30 minutes.